# EUROPEAN PATENT APPLICATION

(11) **EP 1 600 123 A2**
(43) Date of publication of application: **30.11.2005**
(21) Application number: 05011219.2
(22) Date of filing: 24.05.2005
(51) Int. Cl.: A61F 2/06

(54) **Semi-directional drug delivering stents**

(30) Priority: 26.05.2004 US 574898 P
(71) Applicant: Medtronic Vascular, Inc., Santa Rosa, California 95403 (US)
(72) Inventor: Raze, Brian, MN 55304 (US); Tseng, David, Princeton Jct. New Jersey 08550 (US)
(74) Representative: Zimmermann, Gerd Heinrich

(57) **Abstract**

A semi-directional drug delivery stent for selectively delivering one or more therapeutic agents to an area of interest within a bodily or luminal structure is disclosed. In one embodiment, a semi direction drug delivery stent includes a generally cylindrical body defining at least one internal passage positioned longitudinally therein, a non-permeable material applied to the cylindrical body, and at least one therapeutic agent applied to the at least one of the cylindrical body and the non-permeable material.

## Description

### RELATED APPLICATION

The present application claims priority of provisional patent application number 60/574,898, filed on May 26th, 2004.

### FIELD OF THE INVENTION

The present application is directed to a stent configured to deliver one or more therapeutic agents to an area of interest within a bodily or luminal structure. More specifically, a semi-directional drug delivery stent for selectively delivering one or more therapeutic agents to the area of interest within a bodily or luminal structure is disclosed.

### BACKGROUND OF THE INVENTION

The mammalian circulatory system is comprised of a heart, which acts as a pump, and a system of blood vessels which transports blood to various points in the body. For a variety of reasons, the blood vessels and luminal structures associated with the circulatory system may develop a variety of vascular disabilities or dysfunctions. For example, one common vascular dysfunction, commonly known as an aneurysm, is the abnormal widening of the blood vessel. Typically, aneurysms are formed as a result of the weakening of the wall of a blood vessel and subsequent ballooning of the weakened vessel wall. In contrast, stenosis is the narrowing of a lumen or an opening that occurs in organs, vessels, or other luminal structures within the body, thereby impeding or otherwise restricting the flow of blood therethrough. A number of physiological complications have been associated with vascular disabilities or dysfunctions, such as ischemia cardiomyopathy, angina pectoris, and myocardial infarction. In response, several procedures have been developed for treating vascular disabilities or dysfunctions.

One common method used to treat vascular dysfunctions requires the implantation of mechanical support devices, commonly referred to as "stents." Stents act as radially expandable mechanical scaffolds providing support to the incompetent vascular region. In addition, the stent may be coated with one or more therapeutic agents thereby providing a drug-eluding device capable of delivering a therapeutic agent to an area of interest, such as a luminal wall, within a vascular structure. One or more grafts may be positioned on the stent to augment the supportive effects of the stent or to enhance the therapeutic effects of the stent. While stents and stent-graft devices have proven successful in treating a number of vascular dysfunctions, a number of shortcomings have been identified. For example, the targeted delivery of therapeutic agents to areas of interest within luminal structures has proven problematic. More specifically, current drug-eluding stents or stent-graft devices lack the capability to directionally deliver therapeutic agents to an area of repair. As a result, the drug or other therapeutic agent positioned on or otherwise applied to a stent are indiscriminately dispensed into the luminal vessel and bloodstream of a patient. As a general rule, the amount of therapeutic agent loaded on a stent is minute and does not reach systemic, toxic, or physiological concentrations. Consequently, drug delivery stent designers have focused on controlling release of the drug such that localized therapeutic levels are reached and have largely ignored limiting systemic exposure. However, restricting the diffusion of chemotherapeutics into systemic circulation becomes increasingly more important as more cytotoxic agents are used and/or larger drug-eluding vascular prosthetics are employed.

In light of the foregoing, there is an ongoing need for stents and stent-graft devices capable of directionally delivering one or more therapeutic agents to an area within a vascular structure thus minimizing systemic exposure and maximizing local therapeutic effect. It is therefore an object of the present invention to provide an improved stent or stent-graft device which overcomes at least partially the problems associated with the prior art.

### BRIEF SUMMARY OF THE INVENTION

This object is solved by a device for implantation in within a luminal body according to claim 1 and 18, as well as a method of manufacturing a stent according to claim 19 and a method according to claim 20. In accordance with the present invention a semi-directional drug delivery stent for selectively delivering one or more therapeutic agents to an area of interest within a bodily or luminal structure is disclosed.

In one embodiment, a semi-direction drug delivery stent includes a generally cylindrical body defining at least one internal passage positioned longitudinally therein, a non-permeable material applied to the cylindrical body, and at least one therapeutic agent applied to the at least one of the cylindrical body and the non-permeable material. The non-permeable material is configured to act as a diffusion barrier. In one embodiment, the non-permeable material prevents the therapeutic agent from diffusing into the internal passage formed in the stent, thereby effectively preventing the systemic administration of the therapeutic agent through the bloodstream and while delivering the therapeutic agent to tissue positioned proximate to the stent.

In another embodiment, a device for implantation in within a luminal body is disclosed and includes a cylindrical body defining an internal passage formed longitudinally therein, a non-permeable material applied to an outside surface of the cylindrical body, and at least one therapeutic agent applied to the non-permeable material.

A method of making a stent is also disclosed and includes providing a cylindrical body defining a longitudinal internal passage, applying a non-permeable material to an outside surface of the cylindrical body, and applying at least one therapeutic agent to the non-permeable material.

In another embodiment, a method for directionally delivering therapeutic agents to a targeted site within a luminal body is disclosed and includes providing a stent defining a longitudinal internal passage and having a non-permeable material applied to an outside surface of the stent, the non-permeable material having at least one therapeutic agent applied thereto, positioning the stent within a luminal body, eluding the therapeutic agent from the non-permeable material into a wall of the luminal body, and restricting the therapeutic agent from eluding into the internal passage of the stent with the non-permeable material.

### BRIEF DESCRIPTION OF THE DRAWINGS

Various embodiments of a semi-directional drug delivery stent will be explained in more detail by way of the accompanying drawings, wherein components having similar but not necessarily the same or identical features, may have the same reference numeral, and wherein:

Fig. 1 shows a perspective view of an embodiment of a radially expandable stent;

Fig. 2 shows a perspective view of an embodiment of a semi-directional drug delivery stent having a non-permeable graft positioned thereon;

Fig. 2A shows a perspective view of an embodiment of the semi-directional drug delivery stent wherein the non-permeable graft positioned thereon includes one or more surface irregularities formed thereon;

Fig. 3 shows a cross sectional view of an embodiment of a semi-directional drug delivery stent as viewed along lines 4A-4A of Fig. 2;

Fig. 4 shows a cross sectional view of an embodiment of a semi-directional drug delivery stent as viewed along lines 4B-4B of Fig. 2;

Fig. 5 shows a cross sectional view of another embodiment of a semi-directional drug delivery stent as might be viewed along lines 4A-4A of Fig. 2 in a stent graft similar to the embodiment shown in Fig. 2,

Fig. 6 shows a cross sectional view of an embodiment of a semi-directional drug delivery stent implanted within a luminal structure;

Fig. 7 shows a perspective view of an embodiment of a semi-directional drug delivery stent having a non-permeable graft with a sealing layer positioned thereon; and

Fig. 8 shows a perspective view of an embodiment of the semi-directional drug delivery stent wherein the stent includes a distal portion having a diameter greater than the diameter of a medial portion.

### DETAILED DESCRIPTION

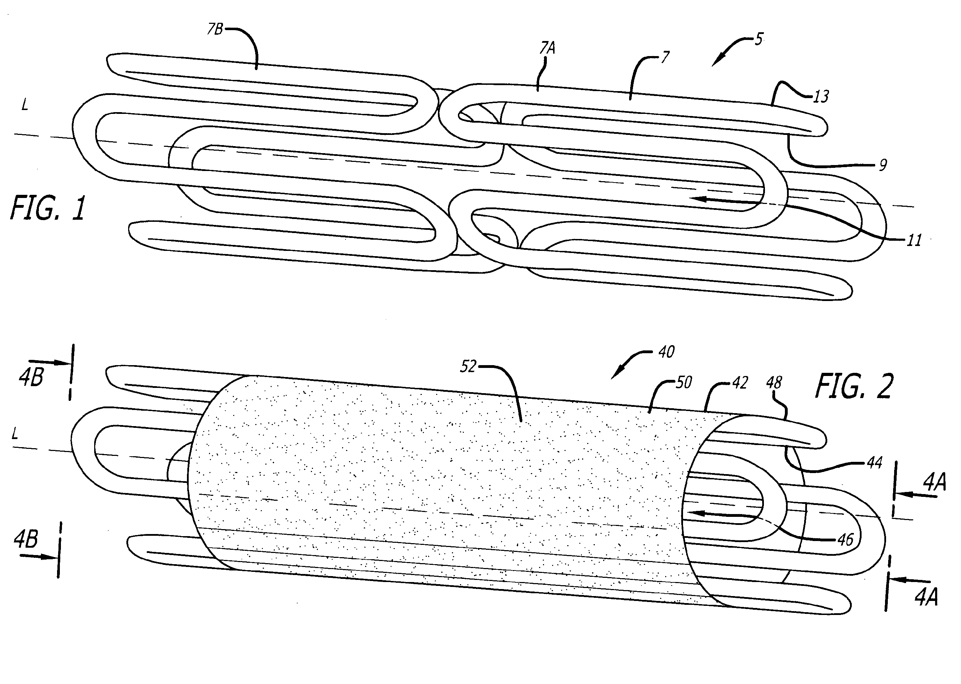

Fig. 1 shows an embodiment of a radially expandable stent configured to be inserted into a luminal structure. As shown, the stent 5 comprises a cylindrical body 7 having an internal surface 9 defining an internal passage 11, and an outer surface 13. The internal passage 11 is coaxially positioned along the longitudinal axis L of the stent 5. In the illustrated embodiment, the stent 5 is comprised of a first cylindrical body member 7A coupled to a second cylindrical body member 7B, thereby forming a modular radially expandable stent. In another embodiment, any number of cylindrical body members may be coupled together to form a modular radially expandable stent. Optionally, the stent 5 may be comprised of a singular cylindrical body 7. The radially expandable stent 5 may be manufactured in a variety of sizes, lengths, and diameters (inside diameters as well as outside diameters). Furthermore, the radially expandable stent 5 may be manufactured from a variety of materials, including, without limitation, stainless steel, tantalum, titanium, nickel-titanium alloys, shape-memory alloys, super elastic alloys, low-modulus Ti-Nb-Zr alloys, cobalt-nickel alloy steel (MB-35N), biologically compatible polymers and elastomers, including non-porous, porous, and micro-porous polymers and elastomers.

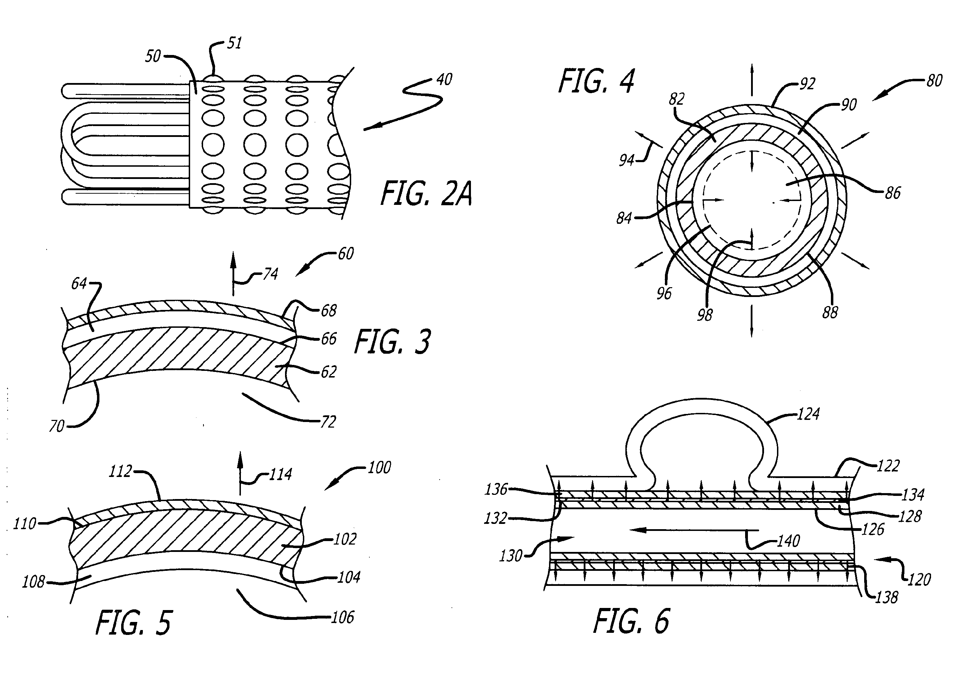

Fig. 2 shows an embodiment of a semi-directional drug delivery stent/stent graft. As shown in Fig. 2, the semi-directional drug delivery stent 40 includes a cylindrical body 42 having an internal surface 44 defining an internal passage 46, and an outer surface 48. Like the previous embodiments, the internal passage 46 is coaxially positioned along the longitudinal axis L of the semi-directional drug delivery stent 40. At least one non-permeable membrane or graft 50 may be selectively applied to the internal surface 44, the outer surface 48, or both surfaces. In one embodiment, the graft 50 is manufactured from paralyene. Optionally, the graft 50 may be manufactured from any variety of biologically compatible materials, including, without limitation, polyurethane, paralyene, polyester, Teflon, polypropylene, polyethylene, polyamides, polycarbonate, poly-methyl-methacrylate, poly-butyl-methacrylate, polyvinyl alcohol, polyvinyl acetate, silicone elastomer, polytetrafluoroethylene, polyacrylonitrile, polyvinyl chloride, polystyrene, and polyvinyl propylene

In the embodiment illustrated in Fig. 2 the non-permeable graft 50 is applied to the outer surface 48 of the semi-directional drug delivery stent 40 and forms a continuous structure. Optionally, the non-permeable graft 50 may be fenestrated or include one or more surface irregularities thereon. For example, the non-permeable graft 50 may include one or more openings or slits formed thereon. In an alternate embodiment, the non-permeable graft 50 includes one or more attachment bumps or similar surface irregularities to enhance stent placement and attachment. Fig. 2A shows an embodiment of the semi-directional drug delivery stent 40 wherein the non-permeable graft 50 includes one or more bumps 51 formed on the outer surface of the non-permeable graft 50. In one embodiment, the one or more bumps may be formed on the non-permeable graft 50 during the manufacture thereof. Other therapeutic agents that could be used in the treating aneurysms with an embodiment of the present invention include: ACE inhibitor/ARB combination therapy; Doxycycline, and other MMP inhibitors (i.e., tetracycline); COX-2 inhibitors; Cerivastatin; Oleic acid; Selective iNOS inhibitor (ON1714, BBS-2); Roxithromycin; Curcumin, gingerol; Beta blockers (cardioselective or carvedilol); and NSAIDS.

Referring again to Fig. 2, at least one therapeutic agent 52 may be applied to or otherwise disposed on the non-permeable graft 50. Exemplary non-permeable graft 50 may be manufactured from a variety of materials. In one embodiment, when a hydrophilic therapeutic agent is being delivered to an area of interest within a body, a hydrophobic membrane or coating may be applied to a graft to prevent the diffusion of drug through the graft. Exemplary hydrophobic materials include, without limitation, polyurethane, polytetrafluoroethylene, fluoro base materials. polyethylene, polypropylene, polyamide, silicon, polydimethylsiloxane, silicon based materials, or a blend or alloy of the above materials. In an alternate embodiment, when a hydrophobic drug is to be delivered, either a hydrophilic and/or a hydrophobic graft or coating may be applied to the stent to prevent the diffusion of drug through the graft, as hydrophilic materials can efficiently block the diffusion of drug. In addition, the hydrophobic coating can be used to block the diffusion of blood (with drug in it) thought the graft. Exemplary hydrophilic materials may include, without limitation, polyvinyl alcohol, poly-ethylene-co-vinyl alcohol, poly vinyl pyrrolidone or a blend or alloy of the above materials. Optionally, the non-permeable graft 50 may also include, without limitation, paralyene, Gore-Tex, or a like material. Those skilled in the art will appreciate that the at least one therapeutic agent 52, the non-permeable material 50, or both may be applied to the cylindrical body 42 in any number of ways, including, without limitation, sprayed, dipped, adhesively bonded, mechanically bonded, and vapor deposited. In an alternate embodiment, one or more therapeutic agents 52 may be applied to the internal surface 44, the outer surface 48, or the internal and outer surface 44, 48 of the semi-directional drug delivery stent 40. As a result, the semi-directional drug delivery stent 40 is capable of eluding or delivering at least one therapeutic agent 52 to an internal passage formed within a luminal structure, to the vessel wall located proximate to the outer surface 48, or both.

The term therapeutic agent as used herein means any component for use in animals having a desired effect. Non-limiting examples include, without limitation, paralyene, anticoagulants, such as an RGD peptide-containing compound, heparin, antithrombin compounds, platelet receptor antagonists, anti-thrombin antibodies, anti-platelet receptor antibodies, aspirin, protaglandin inhibitors, platelet inhibitors, or tick anti-platelet peptide. Other classes of agents include vascular cell antiproliferative agents, such as a growth factor inhibitor, growth factor receptor antagonists, transcriptional repressor or translational repressor, antisense DNA, antisense RNA, replication inhibitor, inhibitory antibodies, antibodies directed against growth factors, cytotoxic agents, cytoskeleton inhibitors, peroxisome proliferator-activated receptor gamma agonists, molecular chaperone inhibitors and bifunctional molecules. The therapeutic agents can also include cholesterol-lowering agents, vasodilating agents, and agents which interfere with endogenous vasoactive mechanisms. Other examples of agents can include anti-inflammatory agents, anti-platelet or fibrinolytic agents, anti-neoplastic agents, anti-allergic agents, anti-rejection agents, metaloprotease inhibitors, anti-microbial or anti-bacterial or anti-viral agents, hormones, vasoactive substances, anti-invasive factors, anti-cancer drugs, antibodies and lymphokines, anti-angiogenic agents, radioactive agents and gene therapy drugs, among others.

Specific non-limiting examples of agents that fall under one or more of the above categories include paclitaxel, docetaxel and derivatives, epothilones, nitric oxide release agents, heparin, aspirin, coumadin, D-phenylalanyl-prolyl-arginine chloromethylketone (PPACK), hirudin, polypeptide from angiostatin and endostatin, benzoquinone ansamycins including geldanamycin, herbimycin and macbecin, methotrexate, 5-fluorouracil, estradiol, P-selectin Glycoprotein ligand-1 chimera, abciximab, exochelin, eleutherobin and sarcodictyin, fludarabine, sirolimus, rapamycin, ABT-578, certican, Sulindac, tranilast, thiazolidinediones including rosiglitazone, troglitazone, pioglitazone, darglitazone and englitazone, tetracyclines, VEGF, transforming growth factor (TGF)-beta, insulin-like growth factor (IGF), platelet derived growth factor (PDGF), fibroblast growth factor (FGF), RGD peptide, estrogens including 17 beta-estradiol and beta or gamma ray emitter (radioactive) agents, and various marking agents including radio-opaque, echogenic, and magnetically resonating materials.

Referring again to Fig. 2, the therapeutic agent 52 located on the non-permeable graft 50 of the semi-directional drug delivery stent 40 is restricted or otherwise prevented from diffusing into the internal passage 46 formed therein by the non-permeable characteristics of the non-permeable material 50. Those skilled in the art will appreciate that the non-permeable material may be applied to the internal surface 44, outer surface 48, or both the internal and outer surfaces 44, 48, respectively, of the cylindrical body 40. In an alternate embodiment, at least one therapeutic agent may be applied to the cylindrical body of the semi-directional drug delivery stent prior to the application of the non-permeable material. For example, paralyene may be applied the stent prior to or following the application of the non-permeable material.

Fig. 3 shows a detailed cross-sectional view of an embodiment of the cylindrical body. As shown, the body segment 60 includes a cylindrical body portion 62 having a non-permeable material 64 applied to an outer surface 66 thereof. At least one therapeutic agent 68 may be selectively applied to the non-permeable material 64. The internal surface 70 of the cylindrical body portion 62 defines an internal lumen section 72. As shown in Fig. 3, the therapeutic agent 68 located on the non-permeable material 64 is dispensed in outward direction as shown by arrow 74 from the cylindrical body portion 62. More specifically, the non-permeable material 64 prevents the therapeutic agent from being dispensed within the internal lumen section 72, thereby directionally delivering the therapeutic agent 68. When positioned within a luminal structure, the therapeutic agent 68 will be directionally dispensed into tissue in contact with or positioned proximate to the body segment 60.

Fig. 4 shows a cross-sectional view of an embodiment of semi-directional drug delivery stent. As shown, the stent 80 includes a cylindrical body 82 having an internal surface 84 defining an internal passage 86, and an outer surface 88 having a non-permeable material 90 applied thereto. At least one therapeutic agent 92 may be selectively applied to the non-permeable material 90. The therapeutic agent 92 positioned on the non-permeable material 90 will be directionally eluded outwardly from the stent 80 as illustrated by arrows 94. Optionally, at least one therapeutic agent 96 may be applied to the cylindrical body 82. For example, at least one therapeutic agent 96 may be applied to the internal surface 84 of the cylindrical body 82 thereby permitting the therapeutic agent 96 to be dispensed into the internal passage 86 as illustrated by arrows 98. As a result, the semi-directional drug delivery stent 80 may be used to directionally deliver a therapeutic gauge into surrounding vascular tissue or vessel walls, into the vessel lumen, or both simultaneously if desired. An alternate embodiment, at least one therapeutic agent 92 may be applied to the surrounding vascular tissue or vessel walls while an alternate therapeutic agent 96 is delivered into the bloodstream of a patient.

Fig. 5 shows an alternate embodiment of a cylindrical body section 100 of the semi-directional drug delivery stent. As shown in Fig. 5, the cylindrical body section 100 includes a cylindrical body portion 102 having an internal surface 104 defining an internal lumen section 106. At least one non-permeable material 108 may be selectively applied to the internal surface 104 of the cylindrical body portion 102 and located within the internal lumen section 106. The cylindrical body portion 102 further includes an outer surface 110 having at least one therapeutic agent 112 selectively applied thereto. Like the previous embodiments, the non-permeable material 108 directionally restricts the disbursement of the therapeutic agent 112 as illustrated by arrow 114.

Fig. 6 shows a side cross-sectional view of a semi-directional drug delivery stent 120 positioned within a luminal structure 122. As shown, the luminal structure 122 includes an aneurismal sac 124. The stent 120 includes a cylindrical body 126 having an internal surface 128 defining an internal passage 130 therethrough. Further, the outer surface 132 of the cylindrical body 126 includes a non-permeable material 134 applied thereto. At least one therapeutic agent 136 may be applied to the stent 120. In the illustrated embodiment, the therapeutic agent 136 is applied to an outer surface of the non-permeable material 134. However, the therapeutic agent 136 may be applied to the internal surface 128, the outer surface 132, the non-permeable material 134, or any combination thereof. As shown in Fig. 6, the therapeutic agent 136 applied to the outer surface of the non-permeable material 134 is eluded into the walls of the luminal structure 122 and the aneurismal sac 124, thereby directionally delivering the therapeutic agent 136 as illustrated by directional arrow 138. When implanted in a blood vessel, the semi-directional drug delivery stent 120 permits the directed delivery of drugs to a selected area within a luminal structure, while permitting the flow of blood to the internal passage 128 as illustrated by directional arrow 140.

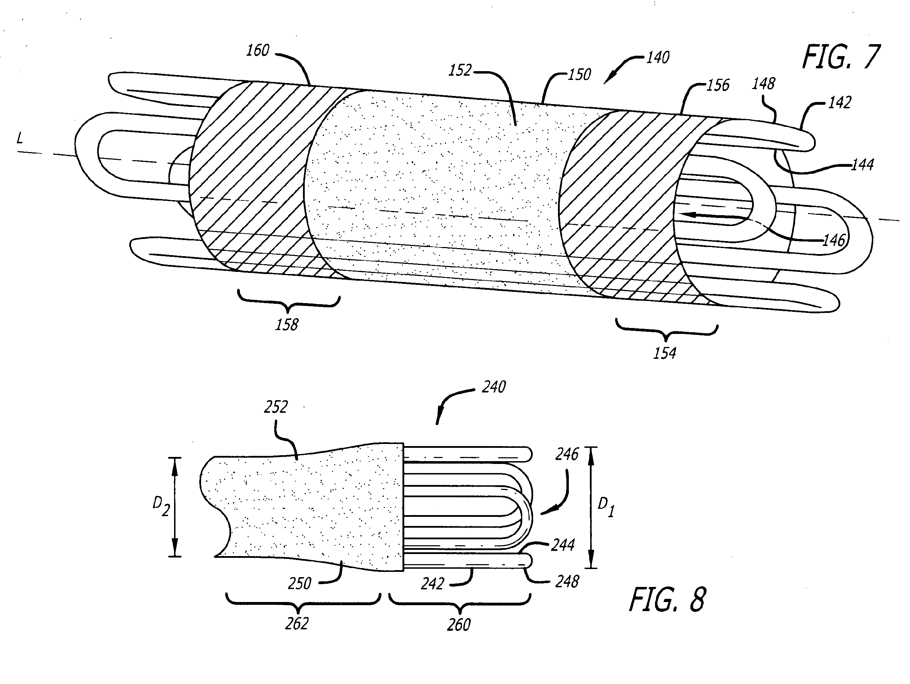

Optionally, the semi-directional drug delivery stent/stent graft may include one or more materials applied thereto or one or more areas formed thereon configured to restrict or prevent the flow of blood through a space between the stent/stent graft and the vessel wall when implanted. As such, the semi-directional drug delivery stent/stent graft may be configured to restrict or prevent endovascular leakage around the stent/stent graft once implanted within a vascular region. For example, Fig. 7 shows an embodiment of a semi-directional drug delivery stent 140 comprising a cylindrical body 142 having an internal surface 144 defining an internal passage 146, and an outer surface 148. Like the previous embodiments, the internal passage 146 is coaxially positioned along the longitudinal axis L of the semi-directional drug delivery stent 140. At least one non-permeable membrane or graft 150 having one or more therapeutic agents 152 applied thereto is selectively applied to the outer surface 148 of the cylindrical body 142. At least one sealing layer may be selectively applied to the stent membrane or graft 150. In the illustrated embodiment a first sealing layer 156 is positioned on a first portion 154 of the graft 150, and a second sealing layer 160 is positioned to a second portion 158 of the graft 150. Optionally, any number of sealing layers may be applied to the graft 150 at various locations. During use, the sealing layers 156, 160 may be configured to provide an essentially fluid-tight seal over at least a circumferential portion of the stent/stent graft. Optionally, the sealing layer may be applied to a portion of the stent/stent graft less than the entire stent/stent graft. In the alternative, the sealing layer may be applied to the entire stent/stent graft. Exemplary materials useful in forming the sealing layer include, without limitation, hydrogels, non-permeable materials, and the like. For example, United States Patent No. 6,656,214, issued to Fogarty et al, which is hereby incorporated by reference in its entirety herein, describes various methods and devices useful in preventing endovascular leakage around an implanted device. More specifically, Col. 6, lines 35 through Col. 8, line 41 describes configurations and materials useful in preventing endovascular leakage which may be applied to the stent/stent graft of the prevent application.

Fig. 8 shows an alternate embodiment of a semi-directional drug delivery stent/stent graft configured to be implanted within a vascular structure while limiting endovascular leakage therearound. Like the previous embodiment, the stent 240 includes a cylindrical body 242 having an internal surface 244 defining an internal passage 246, and an outer surface 248. At least one non-permeable membrane or graft 250 having one or more therapeutic agents 252 applied thereto is selectively applied to the outer surface 248 of the cylindrical body 242. The stent 240 has a distal portion 260 and a medial portion 262. As illustrated, the distal portion may have a first diameter D1 which is greater than the diameter D2 of the medial portion. As such, the distal portion 260 may sealably engage the vascular lumen when expanded, thereby preventing or limiting the effects of endovascular leakage. Optionally, the distal portion 260 may include one or more sealing layers thereon. Further, any number of devices or other mechanism may be utilized with the various embodiments of the stents/stent grafts disclosed herein to prevent or otherwise restrict endovascular leakage.

In closing, it is understood that the embodiments of the semi-directional drug delivery stent disclosed herein are illustrative of principles of the invention. Other modifications may be employed which are within the scope of the present invention. Accordingly, the semi-directional drug delivery stent is not limited to that precisely as shown and described in the present disclosure.

## Claims

1. A device for implantation in within a luminal body, comprising;
a generally cylindrical body defining at least one internal passage positioned longitudinally therein;
a non-permeable material applied to the cylindrical body; and
at least one therapeutic agent applied to the at least one of the cylindrical body and the non-permeable material.

2. The device of claim 1 wherein the cylindrical body is manufactured from at least one material selected from the group consisting of stainless steel, Titanium, Nickel-Titanium alloys, shape memory alloys,

3. The device of claim 1 or 2 wherein the cylindrical body further comprises at least two modular stent bodies coupled together to form a unitary structure.

4. The device of any of the preceding claims wherein the non-permeable material comprises a biologically compatible material selected from a group consisting of polyurethane, paralyene, polyester, Teflon, polypropylene, polyethylene, polyamides, polycarbonate, poly-methyl-methacrylate, poly-butyl-methacrylate, polyvinyl alcohol, polyvinyl acetate, silicone elastomer, polytetrafluoroethylene, polyacrylonitrile, polyvinyl chloride, polystyrene, and polyvinyl propylene.

5. The device of any of the preceding claims wherein the non-permeable material is selectively applied to a portion of the cylindrical body.

6. The device of any of the preceding claims wherein the non-permeable material is applied to an outside surface of the cylindrical body.

7. The device according to any of claims 1 to 5 wherein the non-permeable material is applied to an inside surface of the cylindrical body.

8. The device of any of the preceding claims wherein the non-permeable material is applied to an entire length of the cylindrical body.

9. The device of any of claims 1 to 7 wherein the non-permeable material is applied to a portion of the cylindrical body less than the entire length of the cylindrical body.

10. The device of any of the preceding claims wherein the non-permeable material comprises a coating applied to the cylindrical body.

11. The device of claim 10 wherein the non-permeable material coating is applied to the cylindrical body using at least one method selected from the group consisting of sprayed, dipped, and vapor-deposited.

12. The device of any of the preceding claims wherein the non-permeable material comprises a membrane applied to the cylindrical body.

13. The device of any of the preceding claims wherein the therapeutic agent is selected from the group consisting of paralyene, anticoagulants, RGD peptide-containing compounds, heparin, antithrombin compounds, platelet receptor antagonists, anti-thrombin antibodies, anti-platelet receptor antibodies, aspirin, protaglandin inhibitors, platelet inhibitors, tick anti-platelet peptide, vascular cell antiproliferative agents, growth factor inhibitors, growth factor receptor antagonists, transcriptional repressor, translational repressor, antisense DNA, antisense RNA, replication inhibitor, inhibitory antibodies, antibodies directed against growth factors, cytotoxic agents, cytoskeleton inhibitors, peroxisome proliferator-activated receptor gamma agonists, molecular chaperone inhibitors, bifunctional molecules, cholesterol-lowering agents, vasodilating agents, agents which interfere with endogenous vasoactive mechanisms, anti-inflammatory agents, anti-platelet, anti-fibrinolytic agents, anti-neoplastic agents, anti-allergic agents, anti-rejection agents, metaloprotease inhibitors, anti-microbial or anti-bacterial, anti-viral agents, hormones, vasoactive substances, anti-invasive factors, anti-cancer drugs, antibodies, lymphokines, anti-angiogenic agents, radioactive agents, gene therapy drugs, paclitaxel, docetaxel, docetaxel derivatives, epothilones, nitric oxide release agents, heparin, aspirin, coumadin, D-phenylalanyl-prolyl-arginine chloromethylketone (PPACK), hirudin, polypeptide from angiostatin, polypeptide from endostatin, benzoquinone ansamycins, geldanamycin, herbimycin, macbecin, methotrexate, 5-fluorouracil, estradiol, P-selectin Glycoprotein ligand-1 chimera, abciximab, exochelin, eleutherobin, sarcodictyin, fludarabine, sirolimus, rapamycin, ABT-578, certican, Sulindac, tranilast, thiazolidinediones, rosiglitazone, troglitazone, pioglitazone, darglitazone, englitazone, tetracyclines, VEGF, transforming growth factor (TGF)-beta, insulin-like growth factor (IGF), platelet derived growth factor (PDGF), fibroblast growth factor (FGF), RGD peptide, estrogens, 17 beta-estradiol, beta gamma ray emitter (radioactive) agents, gamma ray emitter (radioactive) agents, and various marking agents including radio-opaque, echogenic, ACE inhibitor/ARB combination therapy, Doxycycline and other MMP inhibitors (i.e., tetracycline); COX-2 inhibitors, Cerivastatin, Oleic acid, Selective iNOS inhibitor (ON1714, BBS-2), Roxithromycin, Curcumin - gingerol, Beta blockers (cardioselective or carvedilol), NSAIDS and magnetically resonating materials.

14. The device of any of the preceding claims wherein the therapeutic agent is applied to the cylindrical body.

15. The device of any of the preceding claims wherein the therapeutic agent is applied to the non-permeable material.

16. The device of any of the preceding claims wherein the therapeutic agent is applied to the cylindrical body and the non-permeable material.

17. The device of any of the preceding claims wherein at least one therapeutic agent is applied to the cylindrical body and at least one other therapeutic agent is applied to the non-permeable material.

18. A device for implantation in within a luminal body, comprising;
a cylindrical body defining an internal passage formed longitudinally therein;
a non-permeable material applied to an outside surface of the cylindrical body; and
at least one therapeutic agent applied to the non-permeable material.

19. A method of making a stent, comprising:
providing a cylindrical body defining a longitudinal internal passage;
applying a non-permeable material to an outside surface of the cylindrical body; and
applying at least one therapeutic agent to the non-permeable material.

20. A method for directionally delivering therapeutic agents to a targeted site within a luminal body, comprising:
providing a stent defining a longitudinal internal passage and having a non-permeable material applied to an outside surface of the stent, the non-permeable material having at least one therapeutic agent applied thereto;
positioning the stent within a luminal body;
eluding the therapeutic agent from the non-permeable material into a wall of the luminal body; and
restricting the therapeutic agent from eluding into the internal passage of the stent with the non-permeable material.
